(19) Europäisches Patentamt
European Patent Office
Office européen des brevets

(11) **EP 4 570 237 A1**

(12) **EUROPEAN PATENT APPLICATION**

(43) Date of publication:
18.06.2025 Bulletin 2025/25

(21) Application number: 24199691.7

(22) Date of filing: 11.09.2024

(51) International Patent Classification (IPC):
*A61K 9/00* (2006.01)    *A61K 9/70* (2006.01)
*A61K 31/465* (2006.01)    *A61K 47/36* (2006.01)

(52) Cooperative Patent Classification (CPC):
**A61K 9/006; A61K 9/7007; A61K 31/465;**
A61K 47/36

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 12.12.2023 CN 202311703613

(71) Applicant: **Shenzhen Huabao Collaborative
Innovation
Technology Research Institute Co., Ltd.
Shenzhen, Guangdong 518100 (CN)**

(72) Inventors:
• **SUN, Qianhui**
**Shenzhen, Guangdong, 518100 (CN)**
• **LIU, Yanzi**
**Shenzhen, Guangdong, 518100 (CN)**
• **SHA, Lili**
**Shenzhen, Guangdong, 518100 (CN)**
• **ZHANG, Han**
**Shenzhen, Guangdong, 518100 (CN)**
• **LI, Yue**
**Shenzhen, Guangdong, 518100 (CN)**
• **ZHU, Song**
**Shenzhen, Guangdong, 518100 (CN)**
• **RONG, Hui**
**Shenzhen, Guangdong, 518100 (CN)**

(74) Representative: **Herrero & Asociados, S.L.
Edificio Aqua - Agustín de Foxá, 4-10
28036 Madrid (ES)**

(54) **ORAL FILM WITH MODERATE NICOTINE RELEASE AND PREPARATION METHOD THEREFOR**

(57) The present application discloses an oral film with moderate nicotine release and a preparation method thereof. The method includes the following steps: preparing a solution of hydroxypropyl chitosan and a solution of polyanion with a nicotine salt to obtain a mixed solution, adding the mixed solution into the hydroxypropyl chitosan solution to obtain a suspension of nicotine salt nanoparticles, and centrifugally drying to obtain nicotine salt nanoparticles; preparing a film-forming solution, adding the nicotine salt and nicotine salt nanoparticles into the film-forming solution according to a compound ratio, and drying to produce a film, thus obtaining an oral film with moderate nicotine release; and the compound ratio of the nicotine salt and the nicotine salt nanoparticles is 1:(0.5-3). The preparation method of the application is straightforward, and the oral film with moderate nicotine release has good mechanical properties and good sustained release effect

Fig. 1

EP 4 570 237 A1

## Description

### Technical field

The present application belongs to the technical field of oral film, in particular to an oral film with moderate nicotine release and a preparation method therefor.

### Background

Nicotine is an alkaloid found in cigarettes and is an essential component of tobacco. People will get dependent on it. When someone stops smoking, they will experience withdrawal symptoms, which can cause irritation and insomnia for a long time. But nicotine can also be used as a drug to relieve these withdrawal symptoms. However, because gastric acid, enzymes in small intestine and first-pass elimination in liver can quickly disturb the absorption of nicotine, the use of oral nicotine is severely restricted. Currently, there are many forms of nicotine administration on the market, such as transdermal patches, oral sprays, chewing gum, buccal pills, etc., but these methods have specific disadvantages. Transdermal patches may cause skin redness and itching; Oral spray nicotine cannot stay in the blood for lengthy periods of time and must be used frequently. The use of nicotine gum is quite complicated, and inappropriate use will impact nicotine absorption. Orally disintegrating film is an ultra-thin, portable and innovative drug dosage form. After being placed on the patient's tongue or oral mucosa, the film is soaked in saliva, hydrated and decomposed, and the drug is slowly released and absorbed by the mucosa, eliminating the need to drink or chew. Additionally, the drug can be immediately absorbed, increasing its bioavailability. The orally disintegrating film is generally composed of pectin, starch, polyvinyl alcohol, sodium alginate, hydroxypropyl methylcellulose and other polymers, together with a plasticizer and flavoring agent. Orally disintegrating film has a high bioavailability, portability, and few adverse reactions, making it a promising novel drug delivery dosage form with a large market potential. However, there are still some problems with nicotine oral film, such as too rapid nicotine release and insufficient mechanical strength. As a result, it is an urgent research subject to develop a nicotine oral film with moderate nicotine release and stable mechanical strength.

### Summary of the invention

The main purpose of the application is to provide an oral film with moderate nicotine release and a preparation method thereof, aiming at solving the problems in the prior art.

To achieve the aforementioned and other related objects, the present application provides a preparation method for an oral film with moderate nicotine release, which includes the following steps:

S1: preparing a solution of hydroxypropyl chitosan and a solution of polyanion with a nicotine salt to obtain a mixed solution, adding the mixed solution into the hydroxypropyl chitosan solution to obtain a suspension of nicotine salt nanoparticles, and centrifugally drying to obtain nicotine salt nanoparticles;

S2: preparing a film-forming solution, adding the nicotine salt and nicotine salt nanoparticles into the film-forming solution according to a compound ratio, and drying to produce a film, thus obtaining an oral film with moderate nicotine release; and

the compound ratio of the nicotine salt and the nicotine salt nanoparticles is 1: (0.5-3), such as 1: 0.5, 1: 1, 1: 1.5, 1: 2, 1: 2.5, 1: 3, and preferably 1: (1-3).

Hydroxypropyl chitosan, as an excellent nano-material, is a hydroxypropyl modification of chitosan. The addition of hydroxypropyl enhances the solubility of chitosan and provides the benefits of low toxicity, good biocompatibility, and non-toxic breakdown products in vivo. According to the present application, hydroxypropyl chitosan is used as a carrier, polyanion is used as a cross-linking agent, nicotine salt nanoparticles are prepared by an ionic gel method, and then nicotine salt and nicotine salt nanoparticles are compounded and added to an oral film to realize a moderate-release effect on nicotine. Moreover, the addition of nicotine salt nanoparticles can also significantly enhance the mechanical properties of the oral film. This is because the action of the nicotine salt nanoparticles on the film matrix helps to increase the mechanical strength of the oral film. When utilizing an oral film, users can adjust its position. And, oral films with high mechanical strength can be mass-produced in industry and stored more effectively. All of these benefits can be combined to lessen the financial loss that results from the breakage of oral film during product shipping. As a result, adding nicotine salt nanoparticles to the oral film provides two benefits: it extends the peak time of nicotine release in the oral film while also increasing its physical and mechanical properties. In order to give users the best experience, nicotine salt and nicotine salt nanoparticles can be compounded in different ratios. This allows the nicotine salt to be released quickly from the oral film to satisfy users, and the nicotine salt nanoparticles to release nicotine continuously and slowly over time to eventually reach a moderate release of nicotine. The optimal release effect of nicotine from the oral film can be achieved when the compound ratio is 1: (1-3).

Furthermore, in step S1, the polyanion is selected from at least one of sodium tripolyphosphate, carboxymethyl-β-cyclodextrin and sulfobutyl-β-cyclodextrin. Because hydroxypropyl chitosan is positively charged and polyanion is negatively charged, they can encapsulate nicotine salt by electrostatic attraction.

Furthermore, the nicotine salt is selected from at least one of nicotine monotartrate, nicotine bitartrate, nicotine hydrochloride, nicotine citrate and nicotine malate.

Furthermore, a mass ratio of hydroxypropyl chitosan to polyanion is (4-6): 1. Hydroxypropyl chitosan and polyanion are used as wall materials to encapsulate the core material nicotine salt. If the mass ratio exceeds this range, the encapsulation efficiency will be affected to some extent.

Furthermore, in step S1, magnetic stirring is used, the stirring speed is 800-1000 r/min , such as 800, 820, 850, 880, 900, 950 and 1000 r/min, and the stirring time is 10-15 min, such as 10, 11, 12, 13, 14 and 15. Proper stirring speed can guarantee that nanoparticles are encapsulated efficiently.

Furthermore, in step S1, a pH value of the hydroxypropyl chitosan solution is 4.2-5.0, such as 4.2, 4.3, 4.4, 4.5, 4.6, 4.7, 4.8, 4.9 and 5.0. The purpose of keeping pH slightly acidic is to better dissolve hydroxypropyl chitosan. Also, the encapsulation efficiency of nanoparticles can be adjusted by altering the pH value.

Furthermore, in step S1, a concentration of the hydroxypropyl chitosan solution is 0.5-1.5 mg/mL, such as 0.5, 0.6, 0.7, 0.8, 0.9 and 1.0 mg/mL.

Furthermore, a concentration of the polyanion solution is 1.0-1.5 mg/mL, such as 1.0, 1.1, 1.2, 1.3, 1.4 and 1.5 mg/mL.

The present application also provides an oral film with moderate nicotine release, prepared according to above-described method. The oral film contains a total of 2.0-4.0 mg of nicotine per tablet, such as 2.0, 2.5, 3.0, 3.5 and 4.0 mg/tablet, and the size of each tablet is 1.0 cm×3.0 cm.

In comparison to the prior art, the embodiments of the application have the following beneficial effects.

When used, the oral film provided by the application can release nicotine into the oral cavity gradually and at a reasonably moderate rate, which helps it overcome the drawbacks of conventional oral films, which include an excessively large nicotine release amount in a short period of time and an excessively rapid nicotine release rate. Additionally, the application-prepared oral film has strong mechanical properties, allowing it to satisfy both consumer and industrial mass production needs.

## Brief description of the drawings

In order to explain the technical solution of the embodiments of this application more clearly, the drawings described in the description of the embodiments of this application will be briefly introduced below. Obviously, the drawings in the present application and their accompanying detailed description are directed to merely exemplary embodiments of the application. For those of ordinary skill in this field, other drawings may be obtained according to these drawings without any creative effort.

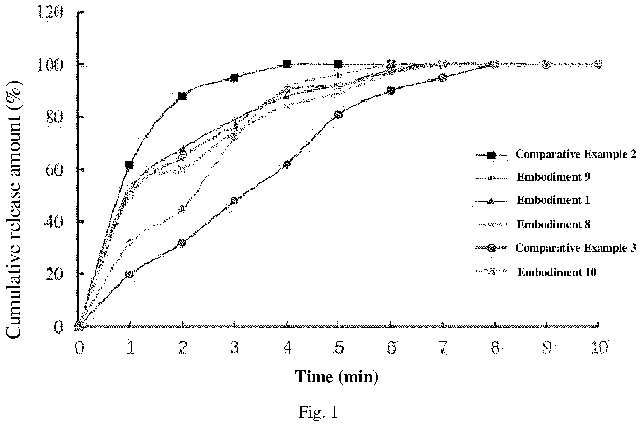

Fig. 1 is a graph showing the results of nicotine release of an oral film, which was prepared according to nicotine salt and nicotine salt nanoparticles with different compound ratios.

## Detailed description of preferred embodiments

To help those skilled in the art comprehend the solution of the present application, the technical solutions in the embodiments of this application will be clearly and completely detailed below, with reference to the drawings mentioned in the embodiments. Obviously, the described embodiments are merely part of the embodiments of this application, not all of them. Based on the embodiments in this application, all other embodiments obtained by those of ordinary skill in the art without creative effort belong to the protection scope of this application.

### Embodiments

A. The material sources of Embodiments and Comparative Examples

1. All of the drugs used in the application are commercially available.
2. The film-forming solution was prepared using the conventional method: 1.8 g of corn starch was weighed and added to 50 mL of deionized water. The mixture was then stirred in a water bath at 95°C for 20 minutes to gelatinize the starch. The mixture was then cooled to 60°C and 0.9 g of hydroxypropyl methylcellulose (HPMC) was added. The mixture was stirred in the water bath for 20 minutes to obtain solution 1; 0.3 g of sodium alginate (SA) was weighed and added to 50 mL of deionized water. The mixture was then stirred in a water bath at 60°C for 10 minutes, after which 0.3 g of glycerol was added. The mixture was then continuously stirred in the water bath for 20 minutes to obtain solution 2. The aforesaid solutions 1 and 2 were combined and stirred in a water bath at 60°C for 1 hour to create a film-forming solution.

In order to explain the technical solution of the present application, the following description will be made with specific Embodiments.

## Embodiment 1

Step S1: Hydroxypropyl chitosan (HPCS) was weighed and dissolved in ultra-pure water to prepare 0.6 mg/mL HPCS solution, and the pH value was adjusted to 4.5; Sodium tripolyphosphate (TPP) was dissolved in ultra-pure water to prepare a 1.0 mg/mL TPP solution, and 200 mg of nicotine tartrate (ND) was added to 100 mL TPP solution, and the solution was kept in a constant temperature water bath at 50°C for 5 minutes. Under magnetic stirring, the TPP-ND solution was slowly introduced into the HPCS solution (the mass ratio of HPCS to TPP was regulated at 6:1), stirred at 30°C for 15 minutes at 800 r/min to obtain a nanoparticle suspension, then centrifuged at 10000 r/min for 20 minutes. The ND encapsulation efficiency was 77.35%, and the nanoparticles were collected by centrifugation and dried to yield the nicotine salt nanoparticles.

Step S2: Following the film-forming solution preparation, the mixed solution of nicotine salt and nicotine salt nanoparticles with a compound ratio of 1: 1 was added, stirred for 1.5 hours, allowed to stand to eliminate bubbles. And then molding was performed on a casting template (10 cm × 10 cm). It was dried at 50°C to create a film, which was then cut into 1.0 cm × 3.0 cm and stored in the dark.

## Embodiment 2

Step S1: Hydroxypropyl chitosan (HPCS) was weighed and dissolved in ultra-pure water to prepare 1.0 mg/mL HPCS solution, and the pH value was adjusted to 4.5; Carboxymethyl-β-cyclodextrin (CM-β-CD) was dissolved in ultra-pure water to prepare a 1.5 mg/mL TPP solution, and 200 mg of nicotine tartrate (ND) was added to 100 mL CM-β-CD solution, and the solution was kept in a constant temperature water bath at 50°C for 5 minutes. Under magnetic stirring, the ND-CM-β-CD solution was slowly introduced into the HPCS solution (the mass ratio of HPCS to CM-β-CD was regulated at 6:1), stirred at 30°C for 15 minutes at 1000 r/min to obtain a nanoparticle suspension, then centrifuged at 10000 r/min for 20 minutes. The ND encapsulation efficiency was 82.31%, and the nanoparticles were collected and dried to produce nicotine salt nanoparticles.

Step S2: It is the same as step S2 of Embodiment 1, except that the adopted nicotine salt is selected from nicotine bitartrate and nicotine hydrochloride with a mixing ratio of 1: 1.

## Embodiment 3

Step S1: Hydroxypropyl chitosan (HPCS) was weighed and dissolved in ultra-pure water to prepare 1.5 mg/mL HPCS solution, and the pH value was adjusted to 4.5; Sulfobutyl-β-cyclodextrin (SBE-β-CD) was dissolved in ultra-pure water to prepare a 1.2 mg/mL SBE-β-CD solution, and 200 mg of nicotine tartrate (ND) was added to 100 mL SBE-β-CD solution, and the solution was kept in a constant temperature water bath at 50°C for 5 minutes. Under magnetic stirring, the NC-SBE-β-CD solution was slowly introduced into the HPCS solution (the mass ratio of HPCS to SBE-β-CD was regulated at 6:1), stirred at 30°C for 12 minutes at 900 r/min to obtain a nanoparticle suspension, then centrifuged at 10000 r/min for 20 minutes. The ND encapsulation efficiency was 80.56%, and the nanoparticles were collected and dried to produce nicotine salt nanoparticles.

Step S2: It is the same as step S2 of Embodiment 1.

## Embodiment 4

Step S1: Hydroxypropyl chitosan (HPCS) was weighed and dissolved in ultra-pure water to prepare 0.6 mg/mL HPCS solution, and the pH value was adjusted to 4.5; Sodium tripolyphosphate (TPP) was dissolved in ultra-pure water to prepare a 1.0 mg/mL TPP solution, and 200 mg of nicotine tartrate (ND) was added to 100 mL TPP solution, and the solution was kept in a constant temperature water bath at 50°C for 5 minutes. Under magnetic stirring, the TPP-ND solution was slowly introduced into the HPCS solution (the mass ratio of HPCS to TPP was regulated at 4:1), stirred at 30°C for 15 minutes at 800 r/min to obtain a nanoparticle suspension, then centrifuged at 10000 r/min for 20 minutes. The ND encapsulation efficiency was 79.78%, and the nanoparticles were collected and dried to produce nicotine salt nanoparticles.

Step S2: It is the same as step S2 of Embodiment 1.

**Embodiment 5**

Step S1: Hydroxypropyl chitosan (HPCS) was weighed and dissolved in ultra-pure water to prepare 0.6 mg/mL HPCS solution, and the pH value was adjusted to 4.5; Sodium tripolyphosphate (TPP) was dissolved in ultra-pure water to prepare a 1.0 mg/mL TPP solution, and 200 mg of nicotine tartrate (ND) was added to 100 mL TPP solution, and the solution was kept in a constant temperature water bath at 50°C for 5 minutes. Under magnetic stirring, the TPP-ND solution was slowly introduced into the HPCS solution (the mass ratio of HPCS to TPP was regulated at 8:1), stirred at 30°C for 15 minutes at 800 r/min to obtain a nanoparticle suspension, then centrifuged at 10000 r/min for 20 minutes. The ND encapsulation efficiency was 70.35%, and the nanoparticles were collected and dried to produce nicotine salt nanoparticles.
Step S2: It is the same as step S2 of Embodiment 1.

**Embodiment 6**

Step S1: Hydroxypropyl chitosan (HPCS) was weighed and dissolved in ultra-pure water to prepare 0.6 mg/mL HPCS solution, and the pH value was adjusted to 5; Sodium tripolyphosphate (TPP) was dissolved in ultra-pure water to prepare a 1.0 mg/mL TPP solution, and 200 mg of nicotine tartrate (ND) was added to 100 mL TPP solution, and the solution was kept in a constant temperature water bath at 50°C for 5 minutes. Under magnetic stirring, the TPP-ND solution was slowly introduced into the HPCS solution (the mass ratio of HPCS to TPP was regulated at 6:1), stirred at 30°C for 15 minutes at 800 r/min to obtain a nanoparticle suspension, then centrifuged at 10000 r/min for 20 minutes. The ND encapsulation efficiency was 81.27%, and the nanoparticles were collected and dried to produce nicotine salt nanoparticles.
Step S2: It is the same as step S2 of Embodiment 1.

**Embodiment 7**

Step S1: Hydroxypropyl chitosan (HPCS) was weighed and dissolved in ultra-pure water to prepare 0.6 mg/mL HPCS solution, and the pH value was adjusted to 7; Sodium tripolyphosphate (TPP) was dissolved in ultra-pure water to prepare a 1.0 mg/mL TPP solution, and 200 mg of nicotine tartrate (ND) was added to 100 mL TPP solution, and the solution was kept in a constant temperature water bath at 50°C for 5 minutes. Under magnetic stirring, the TPP-ND solution was slowly introduced into the HPCS solution (the mass ratio of HPCS to TPP was regulated at 6:1), stirred at 30°C for 15 minutes at 800 r/min to obtain a nanoparticle suspension, then centrifuged at 10000 r/min for 20 minutes. The ND encapsulation efficiency was 71.29%, and the nanoparticles were collected and dried to produce nicotine salt nanoparticles.
Step S2: It is the same as step S2 of Embodiment 1.

**Embodiment 8**

Step S1: It is the same as step S1 of Embodiment 1.
Step S2: Following the film-forming solution preparation, the mixed solution of nicotine salt and nicotine salt nanoparticles with a compound ratio of 1: 2 was added, stirred for 1.5 hours, allowed to stand to eliminate bubbles. And then molding was performed on a casting template (10 cm × 10 cm). It was dried at 50°C to create a film, which was then cut into 1.0 cm × 3.0 cm and stored in the dark.

**Embodiment 9**

Step S1: It is the same as step S1 of Embodiment 1.
Step S2: Following the film-forming solution preparation, the mixed solution of nicotine salt and nicotine salt nanoparticles with a compound ratio of 1: 0.5 was added, stirred for 1.5 hours, allowed to stand to eliminate bubbles. And then molding was performed on a casting template (10 cm × 10 cm). It was dried at 50°C to create a film, which was then cut into 1.0 cm × 3.0 cm and stored in the dark.

**Embodiment 10**

Step S1: It is the same as step S1 of Embodiment 1.
Step S2: Following the film-forming solution preparation, the mixed solution of nicotine salt and nicotine salt nanoparticles with a compound ratio of 1: 3 was added, stirred for 1.5 hours, allowed to stand to eliminate bubbles. And then molding was performed on a casting template (10 cm × 10 cm). It was dried at 50°C to create a film, which was then cut into 1.0 cm × 3.0 cm and stored in the dark.

**Comparative Example 1**

Step S1: Hydroxypropyl chitosan (HPCS) was weighed and dissolved in ultra-pure water to prepare 0.6 mg/mL HPCS solution, and the pH value was adjusted to 4.5; Sodium tripolyphosphate (TPP) was dissolved in ultra-pure water to prepare a 1.0 mg/mL TPP solution, and the solution was kept in a constant temperature water bath at 50°C for 5 minutes. Under magnetic stirring, the TPP solution was slowly introduced into the HPCS solution (the mass ratio of HPCS to TPP was regulated at 6:1), stirred at 30°C for 15 minutes at 800 r/min to obtain a chitosan nanoparticle suspension, then centrifuged at 10000 r/min for 20 minutes. The nanoparticles were collected and dried to produce nicotine salt nanoparticles.
Step S2: Following the film-forming solution preparation, chitosan nanoparticles were added, stirred for 1.5 hours, and allowed to stand to eliminate bubbles. Then molding was performed on a casting template (10 cm × 10 cm). It was dried at 50°C to create a film, which was then cut into 1.0 cm × 3.0 cm and stored in the dark.

**Comparative Example 2**

Following the film-forming solution preparation, nicotine salt was added, stirred for 1.5 hours, and allowed to stand to eliminate bubbles. Then molding was performed on a casting template (10 cm × 10 cm). It was dried at 50°C to create a film, which was then cut into 1.0 cm × 3.0 cm and stored in the dark.

**Comparative Example 3**

Step S1: It is the same as step S1 of Embodiment 1.
Step S2: Following the film-forming solution preparation, nicotine salt nanoparticles were added, stirred for 1.5 hours, and allowed to stand to eliminate bubbles. Then molding was performed on a casting template (10 cm × 10 cm). It was dried at 50°C to create a film, which was then cut into 1.0 cm × 3.0 cm and stored in the dark.

**Comparative Example 4**

Step S1: It is the same as step S1 of Embodiment 1.
Step S2: Following the film-forming solution preparation, the mixed solution of nicotine salt and nicotine salt nanoparticles with a compound ratio of 1: 5 was added, stirred for 1.5 hours, allowed to stand to eliminate bubbles. And then molding was performed on a casting template (10 cm × 10 cm). It was dried at 50°C to create a film, which was then cut into 1.0 cm × 3.0 cm and stored in the dark.

**Comparative Example 5**

Step S1: It is the same as step S1 of Embodiment 1.
Step S2: Following the film-forming solution preparation, the mixed solution of nicotine salt and nicotine salt nanoparticles with a compound ratio of 1: 0.25 was added, stirred for 1.5 hours, allowed to stand to eliminate bubbles. And then molding was performed on a casting template (10 cm × 10 cm). It was dried at 50°C to create a film, which was then cut into 1.0 cm × 3.0 cm and stored in the dark.

B. Performance test methods

1. Quantitative analysis method for nicotine using HPLC

The external standard method of liquid chromatography was used to quantitatively analyze nicotine. The chromatographic conditions were as follows. Chromatographic column: C18 chromatographic column (250 mm ×4.6 mm, 5 μm); Column temperature: 40°C; Mobile phase: a mixture solution of methanol-0.02mol/L phosphate buffer (pH 6.0) containing 0.2% triethylamine with a volume ratio of 30 :70; Flow rate: 0.6 mL/min; Detection wavelength: 259 nm.

2. Determination of nicotine encapsulation efficiency

After the suspension of nicotine salt nanoparticles was prepared according to the embodiments, it was centrifuged at the speed of 10000 r/min for 20 minutes. The supernatant was taken, and the nicotine content was measured using HPLC.

$$\text{Encapsulation efficiency (\%)} = \frac{\text{Nicotine material input - Nicotine content in the supernatant}}{\text{Nicotine material input}} \times 100$$

3. Detection of mechanical properties (tensile strength, elongation at break) of oral film

The mechanical properties of oral film are related to the intermolecular and intramolecular interaction forces in the network formed in the film. Tensile strength (TS) is determined by measuring the breaking point of a sample under tensile stress, which can be used to describe the strength of oral film as the ability to withstand tensile force. Elongation at break (EB) is the extension of the length of a sample from its original length to the breaking point when it is subjected to external force, which may be used to describe the flexibility of the oral film.

The mechanical properties of the oral film were tested by a physical property analyzer, TXT Plus (SMS Company, UK). A stretching device was adopted, its upper arm was fixed to the force sensor and tightened, its lower arm was fixed to the bottom of the device. The force arm of the device was moved downward so that the upper arm and the lower arm of the stretching device are as close as possible, around 2-3 mm apart, and then the device was re-fixed and tightened. For device calibration, the return distance was set to 20 mm, the return speed was 10 mm/s, and the trigger force was 50 g. The oral film was cut into strips of 1.0 cm×3.0 cm, and both ends were clamped by the stretching device for tensile test. At the beginning of the test, the upper arm ran at a pre-test speed of 1 mm/s, and when the force value reached the trigger force of 50 g, the speed was switched to a test speed of 3 mm/s. With the stretching of the oral film, the force value continued to increase until the oral film broke. The maximum extension resistance F and extension distance ΔL were recorded when the oral film breaks, and each oral film was tested for 3 times.

The tensile strength (TS) and elongation at break (EB) of oral film can be calculated according to the following formula:

$$TS = \frac{F}{b \times d}$$

Where TS is the tensile strength, in MPa; F is the maximum extension resistance, in N; b is the film width, in mm; d is the film thickness, in mm.

$$EB(\%) = \frac{\Delta L}{L} \times 100$$

ΔL is the extension distance, in mm; L is the initial length of the film, in mm.

4. Determination of nicotine release from oral film.

200 ml of artificial saliva with pH of 6.8 was put into each beaker, and kept at 37.0±0.1°C and stirred at 100 rpm. 3 pieces of oral film (1.0 cm×3.0 cm) were put into 3 beakers. 2 mL of sample was accurately collected every 1 minute for 10 minutes. After sampling, 2 mL of artificial saliva was added (in order to simulate the cumulative release of nicotine in the mouth, artificial saliva should be added after sampling). 2 mL of solution was taken and adjusted to 10 mL with artificial saliva. The nicotine content was measured using HPLC, and the relative cumulative release of nicotine was calculated. The curve of release is plotted with the relative cumulative release as the ordinate and time as the abscissa. (Tested according to the current standard of artificial saliva, GB/T18886-2019)

The mechanical properties and nicotine release effect of the oral films of Embodiments 1-10 and Comparative Examples 1-5 were tested according to the above determination methods of tensile strength (TS), elongation at break (EB) and nicotine release. The results are shown in Table 1, Table 2 and Table 3.

**Table 1** Mechanical Properties of Embodiments and Comparative Examples

| | Embodiment 1 | Embodiment 2 | Embodiment 3 | Embodiment 4 | Embodiment 5 | Embodiment 6 | Embodiment 7 | Embodiment 8 | Embodiment 9 | Embodiment 10 |
|---|---|---|---|---|---|---|---|---|---|---|
| Tensile strength TS/MPa | 16.87±3.18 | 17.01±2.58 | 16.94±3.24 | 17.24±3.27 | 15.98±2.54 | 17.31±3.15 | 15.18±1.59 | 18.22±2.65 | 14.58±2.59 | 16.06±1.29 |
| Elongation at break EB/% | 24.09±3.27 | 24.57±2.15 | 24.36±2.19 | 24.67±2.51 | 22.69±1.89 | 24.91±2.37 | 22.61±2.16 | 27.21±4.07 | 21.24±3.48 | 23.57±2.41 |

Table 2 Mechanical Properties of Embodiments and Comparative Examples

|  | Comparative Example 1 | Comparative Example 2 | Comparative Example 3 | Comparative Example 4 | Comparative Example 5 |
|---|---|---|---|---|---|
| Tensile strength TS/MPa | 10.25±2.58 | 10.87±3.41 | 20.45±3.74 | 19.16±2.94 | 12.64±2.87 |
| Elongation at break EB/% | 15.47±2.87 | 16.21±3.14 | 29.14±2.98 | 27.84±2.37 | 20.87±1.62 |

Table 3 Nicotine release effect of Embodiments and Comparative Examples

| Time (min) | Cumulative release amount of nicotine (%) | | | | | | | | | | | | | | |
|---|---|---|---|---|---|---|---|---|---|---|---|---|---|---|---|
| | Embo diment 1 | Embo diment 2 | Embo diment 3 | Embo diment 4 | Embo diment 5 | Embo diment 6 | Embo diment 7 | Embo diment 8 | Embo diment 9 | Embo diment 10 | Compara tive Example 1 | Comp arative Examp le 2 | Comp arative Examp le 3 | Comp arative Examp le 4 | Compa rative Exampl e 5 |
| 1 | 52 | 51 | 50 | 52 | 40 | 53 | 49 | 53 | 32 | 50 | - | 62 | 20 | 26 | 56 |
| 2 | 68 | 70 | 69 | 70 | 59 | 69 | 69 | 60 | 45 | 65 | - | 88 | 32 | 35 | 75 |
| 3 | 79 | 80 | 80 | 81 | 73 | 81 | 79 | 74 | 72 | 77 | - | 95 | 48 | 55 | 82 |
| 4 | 88 | 89 | 90 | 90 | 94 | 90 | 90 | 84 | 91 | 90 | - | 100 | 62 | 67 | 98 |
| 5 | 92 | 91 | 92 | 91 | 98 | 91 | 91 | 89 | 96 | 92 | - | 100 | 81 | 86 | 100 |
| 6 | 98 | 98 | 98 | 97 | 100 | 100 | 100 | 96 | 100 | 97 | - | 100 | 90 | 92 | 100 |
| 7 | 100 | 100 | 100 | 100 | 100 | 100 | 100 | 100 | 100 | 100 | - | 100 | 95 | 98 | 100 |
| 8 | 100 | 100 | 100 | 100 | 100 | 100 | 100 | 100 | 100 | 100 | - | 100 | 100 | 100 | 100 |
| 9 | 100 | 100 | 100 | 100 | 100 | 100 | 100 | 100 | 100 | 100 | - | 100 | 100 | 100 | 100 |
| 10 | 100 | 100 | 100 | 100 | 100 | 100 | 100 | 100 | 100 | 100 | - | 100 | 100 | 100 | 100 |

As can be seen from Table 1 and Table 2, with the increase of the compound ratio of nicotine salt nanoparticles, the TS of oral film increases from 10.87 MPa to 20.45 MPa, and EB increases from 16.21% to 29.14%. The introduction of nicotine salt nanoparticles into the HPMC polymer network results in a decrease in the distance among macromolecular chains, a more compact structure for the oral film, and an increase in the TS of oral film. With the increase of the concentration of nicotine salt nanoparticles, the number of hydrogen bonds in the system increases, and the intramolecular and intermolecular forces increase, which makes the TS of oral film increase and the tensile resistance of the film increase. The increase of EB value shows that the addition of nicotine salt nanoparticles makes the oral film more flexible.

As can be seen from Table 3, the release rate of Comparative Example 2 only added with nicotine oral film in artificial saliva is the fastest, reaching 90% within 3 minutes. When the compound ratio is high, i.e., the content of nicotine salt nanoparticles is low (Comparative Example 5), the release rate of nicotine in artificial saliva is faster, reaching 90% within 3.5min. However, the release rate of the oral film (Comparative Example 3) only added with nicotine salt nanoparticles in artificial saliva is the slowest, reaching 90% within 6 min. When the compound ratio is low, i.e., the content of nicotine salt nanoparticles is high (Comparative Example 4), the release rate of nicotine in artificial saliva is slow, reaching 90% within 5.5 min, which leads to poor user experience.

According to the tests in Table, Table 2 and Table 3, the mechanical properties of the oral film can be improved by adding nicotine salt nanoparticles into the oral film solution. Additionally, mechanical properties and a moderate nicotine release effect can both be taken into account in an oral film that is prepared by selecting a proper compound ratio. The test results for comprehensive mechanical properties and nicotine release show that the oral films with moderate nicotine release prepared by Embodiments 1-10 have clear advantages over Comparative Examples and can effectively meet the high standards of customers and the market.

In order to more visually present the comparison of the release effects of nicotine oral films with different compound ratios, the release data of Embodiment 1 (compound ratio 1: 1), Embodiment 8 (compound ratio 1: 2), Embodiment 9 (compound ratio 1: 0.5), Embodiment 10 (compound ratio 1:3), Comparative Example 2 (nicotine oral film) and Comparative Example 3 (oral film with nicotine salt nanoparticle) are displayed in graphs. Figure 1 is the release results of nicotine oral films with different compound ratios. It can be seen from Figure 1 that the nicotine release of Embodiment 1 and Embodiments 8-10 are relatively moderate. Especially for Embodiment 1 and Embodiment 8, when the compound ratio is 1: (1-3), the cumulative release amount of nicotine in the oral film increases from about 75% to about 95% within 2 min-7 min, and a higher nicotine release amount can be obtained in a longer time. Therefore, nicotine salt and nicotine salt nanoparticles are compounded and introduced to the oral film, with the best release effect obtained at a compound ratio of 1: 1-1: 3.

The oral film with moderate nicotine release provided by the application and its preparation method are described in detail above. In this paper, the principle and implementation of the application are expounded by using specific embodiments. The above embodiments are merely used to illustrate the technical solutions of the present application, rather than limit it. Although the application has been described in detail with reference to the foregoing embodiments, it should be understood by those skilled in the art that it is still possible to modify the technical solutions described in the foregoing embodiments, or equivalently replace some technical features thereof. These modifications and equivalents do not make the nature of the corresponding technical solution deviates from the spirit and scope of the present application, and shall be included in the protection scope of the present application.

**Claims**

1. A preparation method for an oral film with moderate nicotine release, comprising the following steps:

   preparing a solution of hydroxypropyl chitosan and a solution of polyanion with a nicotine salt to obtain a mixed solution, adding the mixed solution into the hydroxypropyl chitosan solution to obtain a suspension of nicotine salt nanoparticles, and centrifugally drying to obtain nicotine salt nanoparticles;
   preparing a film-forming solution, adding the nicotine salt and nicotine salt nanoparticles into the film-forming solution according to a compound ratio, and drying to produce a film, thus obtaining an oral film with moderate nicotine release; and
   the compound ratio of the nicotine salt and the nicotine salt nanoparticles is 1: (0.5-3).

2. The preparation method of claim 1, wherein the compound ratio of the nicotine salt and the nicotine salt nanoparticles is 1: (1-3).

3. The preparation method of claim 1, wherein the polyanion is selected from at least one of sodium tripolyphosphate, carboxymethyl-β-cyclodextrin and sulfobutyl-β-cyclodextrin.

4. The preparation method of claim 1, wherein the nicotine salt is selected from at least one of nicotine monotartrate, nicotine bitartrate, nicotine hydrochloride, nicotine citrate and nicotine malate.

5. The preparation method of claim 1, wherein a mass ratio of hydroxypropyl chitosan to polyanion is (4-6): 1.

6. The preparation method of claim 1, wherein a stirring speed is 800-1000 r/min and a stirring time is 10-15 min.

7. The preparation method of claim 1, wherein a pH value of the hydroxypropyl chitosan solution is 4.2-5.0.

8. The preparation method of claim 1, wherein a concentration of the hydroxypropyl chitosan solution is 0.5-1.5 mg/mL.

9. The preparation method of claim 1, wherein a concentration of the polyanion solution is 1.0-1.5 mg/mL.

10. An oral film with moderate nicotine release, **characterized by** being prepared by the preparation method of any one of claims 1 to 9.

11. The oral film with moderate nicotine release of claim 10, a total content of nicotine in the oral film is 2.0-4.0 mg/ tablet.

Fig. 1

## EUROPEAN SEARCH REPORT

Europäisches
Patentamt
European
Patent Office
Office européen
des brevets

**Application Number**

EP 24 19 9691

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2023/130055 A1 (WARGACKI STEPHEN PAUL [US] ET AL) 27 April 2023 (2023-04-27) | 10,11 | INV. A61K9/00 |
| A | * the whole document * <br> * paragraph [0145] - paragraph [0148]; claims 1-68 * | 1-9 | A61K9/70 <br> A61K31/465 |
| | ----- | | ADD. |
| Y | US 9 675 548 B2 (CHEN LI-LAN [US]; GLAXOSMITHKLINE LLC [US]) 13 June 2017 (2017-06-13) | 10,11 | A61K47/36 |
| A | * the whole document * <br> * claims 1-9 * | 1-9 | |
| | ----- | | |

**TECHNICAL FIELDS SEARCHED (IPC)**

A61K

The present search report has been drawn up for all claims

| Place of search | Date of completion of the search | Examiner |
|---|---|---|
| Munich | 27 January 2025 | Felder, Christian |

EPO FORM 1503 03.82 (P04C01)

## ANNEX TO THE EUROPEAN SEARCH REPORT
## ON EUROPEAN PATENT APPLICATION NO.

EP 24 19 9691

This annex lists the patent family members relating to the patent documents cited in  the above-mentioned European search report.
The members are as contained in the European Patent Office EDP file on
The European Patent Office is in no way liable for these particulars which are merely given for the purpose of information.

27-01-2025

| Patent document cited in search report | Publication date | Patent family member(s) | Publication date |
|---|---|---|---|
| US 2023130055 A1 | 27-04-2023 | CA 3236045 A1 | 27-04-2023 |
| | | CN 118414150 A | 30-07-2024 |
| | | EP 4419088 A1 | 28-08-2024 |
| | | IL 312326 A | 01-06-2024 |
| | | JP 2024540936 A | 06-11-2024 |
| | | KR 20240093850 A | 24-06-2024 |
| | | US 2023130055 A1 | 27-04-2023 |
| | | WO 2023069733 A1 | 27-04-2023 |
| US 9675548 B2 | 13-06-2017 | AR 045076 A1 | 12-10-2005 |
| | | AU 2004259006 A1 | 03-02-2005 |
| | | AU 2010224456 A1 | 21-10-2010 |
| | | BR PI0412752 A | 26-09-2006 |
| | | CA 2533577 A1 | 03-02-2005 |
| | | CL 2008002406 A1 | 02-01-2009 |
| | | CL 2008002407 A1 | 02-01-2009 |
| | | CN 1826117 A | 30-08-2006 |
| | | DK 1648421 T3 | 04-12-2017 |
| | | DK 2446881 T3 | 02-06-2014 |
| | | EP 1648421 A2 | 26-04-2006 |
| | | EP 2446881 A1 | 02-05-2012 |
| | | ES 2466943 T3 | 11-06-2014 |
| | | ES 2654062 T3 | 12-02-2018 |
| | | HK 1169802 A1 | 08-02-2013 |
| | | HR P20171944 T1 | 26-01-2018 |
| | | HU E037508 T2 | 28-09-2018 |
| | | JP 5038477 B2 | 03-10-2012 |
| | | JP 5378270 B2 | 25-12-2013 |
| | | JP 5572133 B2 | 13-08-2014 |
| | | JP 2006528636 A | 21-12-2006 |
| | | JP 2010120975 A | 03-06-2010 |
| | | JP 2011037879 A | 24-02-2011 |
| | | JP 2011225620 A | 10-11-2011 |
| | | LT 1648421 T | 25-01-2018 |
| | | MX PA06000852 A | 30-03-2006 |
| | | PE 20050496 A1 | 12-06-2005 |
| | | PL 1648421 T3 | 30-03-2018 |
| | | PL 2446881 T3 | 29-08-2014 |
| | | PT 1648421 T | 22-12-2017 |
| | | PT 2446881 E | 11-06-2014 |
| | | RU 2351315 C2 | 10-04-2009 |
| | | SI 1648421 T1 | 28-02-2018 |
| | | TW 200514579 A | 01-05-2005 |
| | | US 2006198873 A1 | 07-09-2006 |
| | | UY 28431 A1 | 28-02-2005 |
| | | WO 2005009386 A2 | 03-02-2005 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**

EP 24 19 9691

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2025

| Patent document cited in search report | Publication date | Patent family member(s) | Publication date |
|---|---|---|---|

EPO FORM P0459

For more details about this annex : see Official Journal of the European Patent Office, No. 12/82

page 2 of 2